# EUROPÄISCHE PATENTSCHRIFT

(11) **EP 1 326 526 B1**
(45) Veröffentlichungstag und Bekanntmachung des Hinweises auf die Patenterteilung: **27.12.2006**
(21) Anmeldenummer: 01971471.6
(22) Anmeldetag: 11.10.2001
(51) Int. Cl.: A61B 3/135, A61B 3/14, G02B 21/08, G02B 27/46, G02B 21/00

(54) **ABBILDENDES OPTISCHES GERÄT**
IMAGING OPTICAL DEVICE
APPAREIL OPTIQUE D'IMAGERIE

(30) Priorität: 11.10.2000 DE 10050358
(43) Veröffentlichungstag der Anmeldung: 16.07.2003
(73) Patentinhaber: STEINHUBER, Wolfdietrich, 6080 Igls (AT)
(72) Erfinder: STEINHUBER, Wolfdietrich, 6080 Igls (AT)
(74) Vertreter: Hofinger, Stephan
(86) Internationale Anmeldenummer: PCT/AT2001/000328
(87) Internationale Veröffentlichungsnummer: WO 2002/031571

(56) Entgegenhaltungen:
- US-A- 3 547 512
- US-A- 4 170 398
- US-A- 4 620 318
- US-A- 4 991 953
- US-A- 5 993 002

## Beschreibung

Die vorliegende Erfindung betrifft ein abbildendes optisches Gerät mit einer Lichtquellenanordnung und einem von einem Beobachtungsstrahlengang zumindest abschnittsweise getrennten Beleuchtungsstrahlengang, wobei im Beleuchtungsstrahlengang eine Beleuchtungslinsenanordnung und im Beobachtungsstrahlengang eine Beobachtungslinsenanordnung vorgesehen ist, wobei im Beleuchtungsstrahlengang eine Beleuchtungsfeldblende in einem Abstand von der Beleuchtungslinsenanordnung angeordnet ist, wobei ein abzubildender Gegenstand in der Beleuchtungsbildebene liegt, in die die Beleuchtungslinsenanordnung die Beleuchtungsfeldblende abbildet und wobei die Beobachtungslinsenanordnung den abzubildenden Gegenstand in die Beobachtungsbildebene abbildet.

Gattungsgemäße Geräte sind beim Stand der Technik z.B. aus der WO 97/15855 bekannt. In dieser Schrift sind in den gezeigten Ausführungsbeispielen sowohl im Beobachtungs- als auch im Beleuchtungsstrahlengang Feldblenden zur Begrenzung des Beobachtungs- bzw. Beleuchtungsstrahlenbündels angeordnet. Zur Erzielung einer optimalen Abbildung ist es hierbei nötig, daß sowohl die Feldblendenöffnung im Beobachtungsstrahlengang als auch die Feldblendenöffnung im Beleuchtungsstrahlengang bezüglich der jeweiligen Linsenanordnung in der Bildebene des abzubildenden Gegenstandes angeordnet sind. Durch das gemeinsame Oszillieren der beiden Feldblendenöffnungen wird der zu beobachtende Gegenstand streifenweise optisch abgetastet. Bei entsprechend schneller Bewegung der Blenden ergibt sich ein letztendlich für das menschliche Auge aus den einzelnen abgetasteten Streifen zusammengesetztes Bild.

Allgemein entsteht jede Abbildung durch Sammeln von am Objekt gebeugten Lichtstrahlen. Sie entsteht nach Abbe durch eine zweifache Beugung, wobei die erste durch Beugung der Lichtstrahlen am Objekt und die zweite durch Beugung der Lichtstrahlen durch die abbildende Linse entsteht. Die Linse erzeugt dabei zunächst ein Bild der Lichtquelle in ihrer Brennebene oder in der Bildebene der Lichtquelle und in Folge in der Bildweite die eigentliche Abbildung. Jede Beugung erzeugt Beugungsordnungen. Nach Abbé sind für jede Abbildung mindestens zwei Beugungsordnungen notwendig. Je mehr Beugungsordnungen zur Abbildung beitragen, umso höher wird die Detailauflösung. Das heißt, je größer die Apertur der abbildenden Linsenkombination ist, umso mehr Beugungsordnungen können eindringen und tragen zum Bild bei.

Da die hohen Beugungsordnungen die Linse zunehmend schräger durchlaufen, steigen die Anforderungen an das abbildende Objektiv. Werden die Strahlen nicht annähernd fehlerfrei abgebildet, so verfälschen sie das endgültige Bild. Diese Bildverfälschungen sind bei den gattungsgemäßen beim Stand der Technik bekannten optischen Geräten zu beobachten. Die Abbildung des Lichtes in der Brennebene oder der Bildebene der Lichtquelle ergibt eine charakteristische Anordnung der Beugungsordnungen in der X-Y Richtung, wobei die nullte Beugungsordnung in der Mitte liegt und die höheren Ordnungen sich nach außen anlagern.

Durch Einsetzen einer Maske (Blende) in die Brennebene oder die Bildebene der Lichtquelle, kann in die Bildentstehung in der Bildebene des abzubildenden Gegenstandes eingegriffen werden. Die Blenden in der Brennebene oder der Bildebene der Lichtquelle haben keine bildfeldbegrenzende Funktion. Sie können aber Beugungsordnungen gezielt abblenden, sodaß diese im Endbild nicht mehr vorkommen. Die einfachste Form einer solchen Blende (Raumfilter) stellt eine kreisförmige Blendenöffnung dar. Sie schneidet die äußersten Beugungsordnungen aus dem Bild. Die Anwendung empfiehlt sich, wenn die höchsten Ortsfrequenzen (Beugungsordnungen) nicht zur Abbildung sinnvoll sind, entweder weil z.B. höchste Auflösung nicht erforderlich ist oder weil das Objektiv diese Beugungsordnungen nicht fehlerfrei abbilden kann. Die ausschließliche bisher bekannte Anordnung von Raumfiltern in der hinteren Brennebene oder der Bildebene der Lichtquelle stellt eine konstruktive Einschränkung beim Bau von optischen abbildenden Geräten dar.

Aufgabe der Erfindung ist es somit, ein im Hinblick auf die Abbildungsqualität verbessertes optisches Gerät zu schaffen, bei dem die oben genannten Einschränkungen beseitigt sind.

Dies wird erfindungsgemäß dadurch erreicht, daß mindestens ein, vorzugsweise durch eine Blende gebildeter, Raumfilter im Beobachtungsstrahlengang außerhalb der Beobachtungsbildebene angeordnet ist, wobei der bzw. die Raumfilter und die Beleuchtungsfeldblende sowie eine gegebenenfalls vorhandene Beobachtungsfeldblende in einem festen geometrischen Zusammenhang zueinander - vorzugsweise oszillierend - beweglich gelagert sind.

Die erfindungsgemäße Anordnung von mindestens einem Raumfilter im Beobachtungsstrahlengang dient der Beseitigung von störenden Bildanteilen, welche durch schlecht übertragene höhere Beugungsordnungen hervorgerufen werden und kann grundsätzlich in verschiedenen optischen Geräten, wie z.B. optischen Scannern, Mikroskopen oder Ophthalmoskopen angewendet werden. Der oder die Raumfilter kann (können) dabei an die jeweilige Konstruktion und Aufgabenstellung des optischen Gerätes angepaßt, außerhalb der Beobachtungsbildebene relativ frei und angepaßt an die Anforderungen des jeweiligen optischen Gerätes, angeordnet werden. Hierbei wird insbesondere durch die bewegliche Lagerung des bzw. der Raumfilter(s) sowie der Beleuchtungsfeldblende und einer gegebenenfalls vorhandenen Beobachtungsfeldblende in einem festen geometrischen Zusammenhang zueinander ein Gerät zur Verfügung gestellt, bei dem der abzubildende Gegenstand bereichsweise bzw. streifenweise abgescannt werden kann, wobei gleichzeitig störende Bildanteile beseitigt sind. So kann z.B. durch die erfindungsgemäße Verwendung von Raumfiltern bei einem Ophthalmoskop die dort oft auftretende Rotstichigkeit des in der Beobachtungsbildebene erzeugten Bildes des Gegenstandes beseitigt werden.

Besonders günstig ist die Anordnung der Raumfilter außerhalb der Brennebene der Beobachtungslinsenanordnung. Darüber hinaus sehen bevorzugte Varianten vor, daß mindestens ein Raumfilter außerhalb der Bildebene der Lichtquelle angeordnet ist. Bei Lichtquellen, welche paralleles Licht erzeugen, fallen die Brennebene und die Bildebene der Lichtquelle zusammen.

Eine weitere Verbesserung des Filtereffektes wird durch die Anordnung von zwei oder mehreren Raumfiltern im Beobachtungsstrahlengang erreicht. Hierzu stehen grundsätzlich verschiedene Varianten zur Verfügung. So kann z.B. vorgesehen sein, daß ein Raumfilter vor und ein Raumfilter hinter der Beobachtungsbildebene im Beobachtungsstrahlengang angeordnet sind. In einer anderen Ausführungsform ist wiederum vorgesehen, daß zwei Raumfilter vor oder hinter der Beobachtungsbildebene im Beobachtungsstrahlengang angeordnet sind.

Neben der erfindungsgemäßen Anordnung mindestens eines Raumfilters im Beobachtungsstrahlengang kann wie beim Stand der Technik bereits vorab bekannt, auch vorgesehen sein, daß eine Beobachtungsfeldblende in der Beobachtungsbildebene im Beobachtungsstrahlengang angeordnet ist.

Eine besonders gute Raumfilterwirkung wird dadurch hervorgerufen, daß der oder die Raumfilter in relativer Nähe zur Beobachtungsbildebene angeordnet sind. Die Entfernung zwischen dem (den) Raumfilter(n) und der Beobachtungsbildebene kann hierbei unter Zuhilfenahme der Beobachtungsbildweite definiert werden. Die Beobachtungsbildweite ist hierbei die Entfernung zwischen Beobachtungsbildebene und Beobachtungslinsenanordnung. In einer günstigen Ausführungsform ist es vorgesehen, daß mindestens ein, vorzugsweise alle, Raumfilter im Beobachtungsstrahlengang weniger als 20 %, vorzugsweise weniger als 10 %, der Beobachtungsbildweite von der Beobachtungsbildebene entfernt sind. Darüber hinaus ist es besonders günstig, daß mindestens ein, vorzugsweise alle, Raumfilter als eine Blende mit einer Öffnungsweite zwischen 0,5 % und 1,5 %, vorzugsweise von 1 %, der Beobachtungsbildweite ausgeführt ist (sind).

Neben der Anordnung mindestens eines Raumfilters im Beobachtungsstrahlengang kann zur weiteren Verbesserung des in der Beobachtungsbildebene abgebildeten Bildes des zu beobachtenden Gegenstandes auch vorgesehen sein, daß zumindest ein weiterer Raumfilter vor und/oder hinter der Beleuchtungsfeldblende im Beleuchtungsstrahlengang angeordnet ist. Hierbei werden wiederum günstige Filterwirkungen erzielt, wenn mindestens ein, vorzugsweise alle, weiteren Raumfilter im Beleuchtungsstrahlengang weniger als 20 %, vorzugsweise weniger als 10 %, des Abstandes zwischen Beleuchtungsfeldblende und Beleuchtungslinsenanordnung von der Beleuchtungsfeldblende entfernt sind. Bei der Verwendung von Blenden als Raumfilter im Beleuchtungsstrahlengang ist es darüber hinaus günstig, die selben Öffnungsweiten der Blenden wie bei den im Beobachtungsstrahlengang angeordneten Raumfiltern zu verwenden. Um den bzw. die Raumfilter und die Beleuchtungsfeldblende sowie eine gegebenenfalls vorhandene Beobachtungsfeldblende in einem festen geometrischen Zusammenhang beweglich lagern zu können, ist es vorzugsweise vorgesehen, diese in einem beweglich gelagerten Bauteil zusammenzufassen.

Weitere Merkmale und Einzelheiten der vorliegenden Erfindung ergeben sich aus den nachfolgenden Figurenbeschreibungen. Dabei zeigt
Fig. 1 ein Ophthalmoskop nach dem Stand der Technik,
Fig. 2 eine Prinzipskizze zur erfindungsgemäßen Anordnung mindestens eines Raumfilters,
Fig. 3 ein Ophthalmoskop unter Verwendung einer erfindungsgemäßen Anordnung von oszillierenden Raumfiltern,

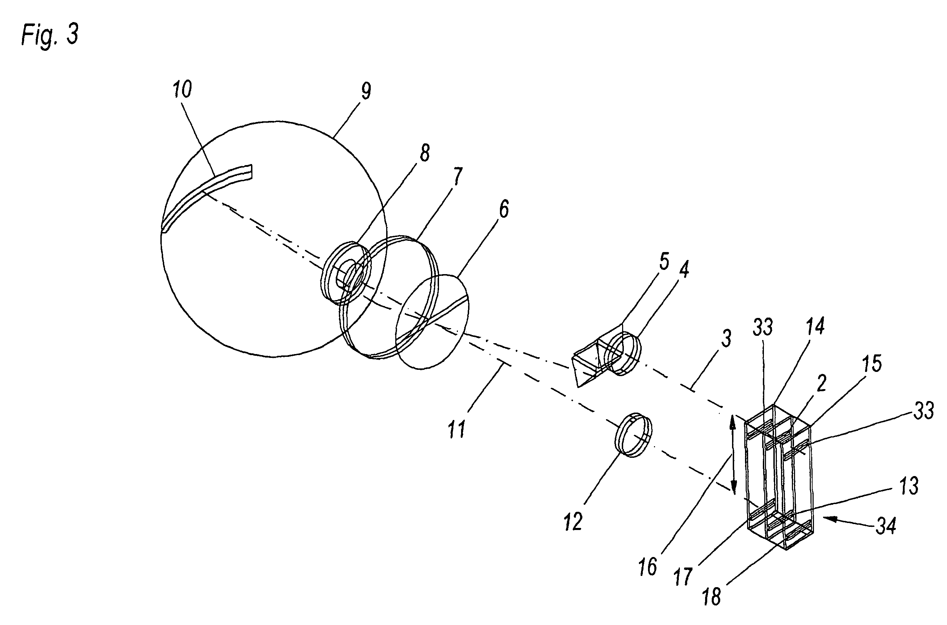

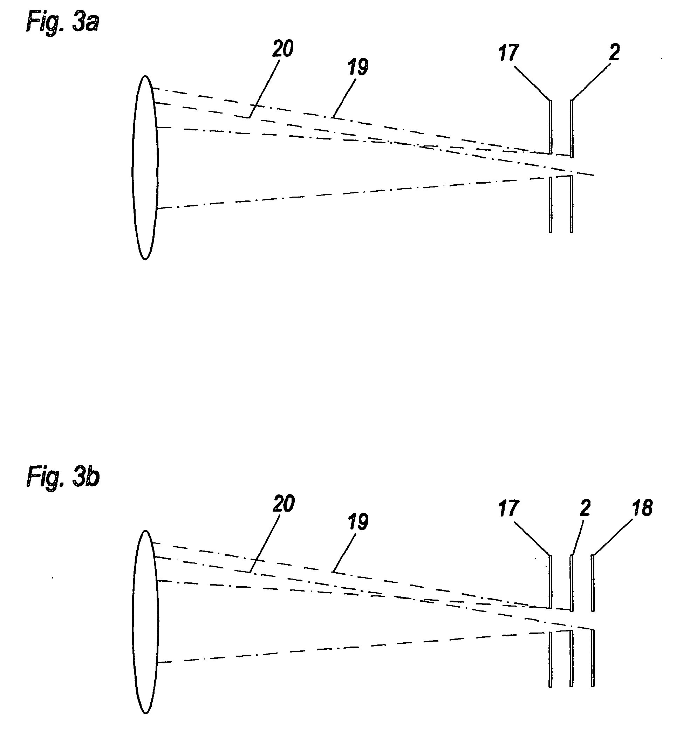

Fig. 3 a einen nur aus einer vorderen Blende bestehenden Raumfilter,
Fig. 3 b einen aus einer vorderen und einer hinteren Blende bestehenden Raumfilter, und

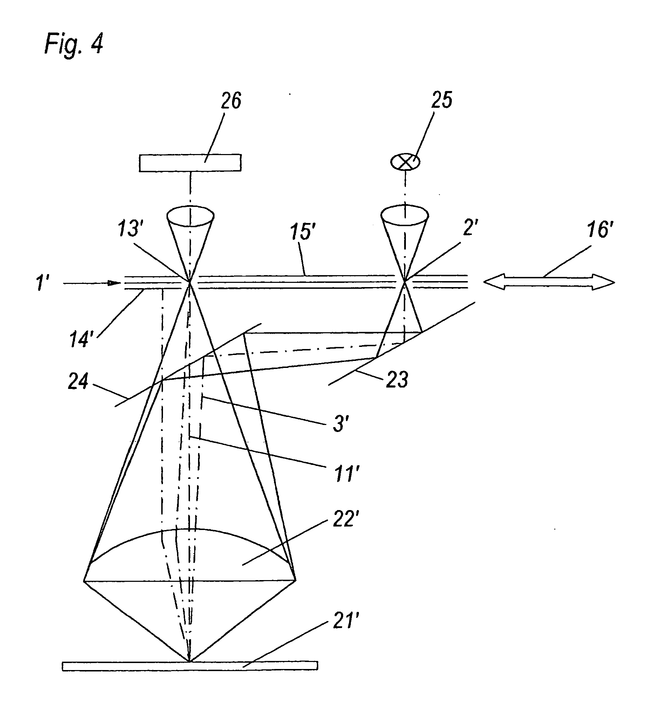

Fig. 4 ein Auflichtmikroskop mit erfindungsgemäßen Raumfiltern.

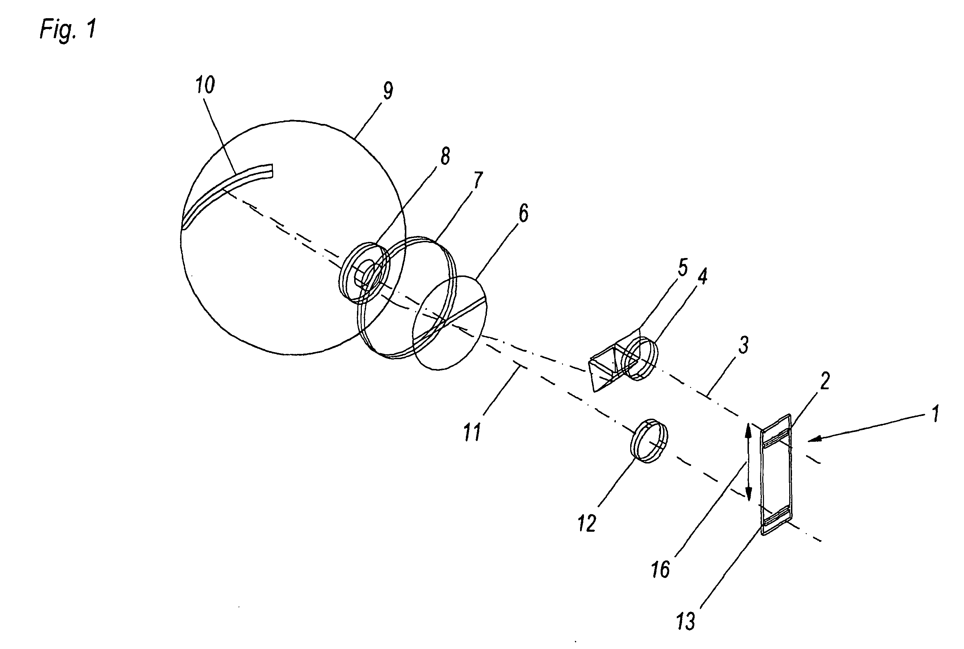

Bei dem in Fig. 1 dargestellten Stand der Technik wird die Retina 10 des Auges 9 mit Hilfe eines Ophthalmoskopes strichweise ausgeleuchtet bzw, abgetastet. Durch die Öffnung der Beleuchtungsfeldblende 2 im Beleuchtungsstrahlengang 3 tritt Licht entlang der Achse des Beleuchtungsstrahlenganges 3 in das Beleuchtungsobjektiv (Beleuchtungslinsenanordnung) 4 und wird im gesteuerten Prismensystem 5 derart abgelenkt, daß es in der Zwischenbildebene 6 mit der Achse des Beobachtungsstrahlenganges 11 zusammenfällt. Im Anschluß an die annähernde Parallelstellung des eintretenden Strahlenbündels in der Ophthalmoskopielupe 7 tritt der Lichtstrahl durch die Pupille 8 in das Auge 9 ein und beleuchtet die dargestellten Lichtstreifen der Retina 10. Das dort gebeugte Licht wird entlang der Achse des Beobachtungsstrahlenganges 11 zurückgeworfen und kann nach Durchlaufen des Beobachtungsobjektives (Beobachtungs linsenanordnung) 12 und der Beobachtungsfeldblende 13 mit einem optischen Sensor (z.B. Auge eines Arztes) aufgenommen werden. Durch das Oszillieren der Doppelblende 1 wird der Lichtstreifen 10 auf der Retina verschoben. In der Doppelblende 1 sind hierbei die Beleuchtungsfeldblende 2 und die Beobachtungsfeldblende 13 in einer Baueinheit zusammengefaßt. Oszilliert nun die Doppelblende 1 in der Bewegungsrichtung 16 mit einer entsprechend hohen Frequenz (z. B. 50 Hertz) so ergibt sich für ein das Licht aus dem Beobachtungsstrahlengang 11 in der Öffnung der Beobachtungsfeldblende 13 aufnehmendes menschliches Auge ein zusammenhängendes Abbild der Retina 10 des beobachteten Auges 9.

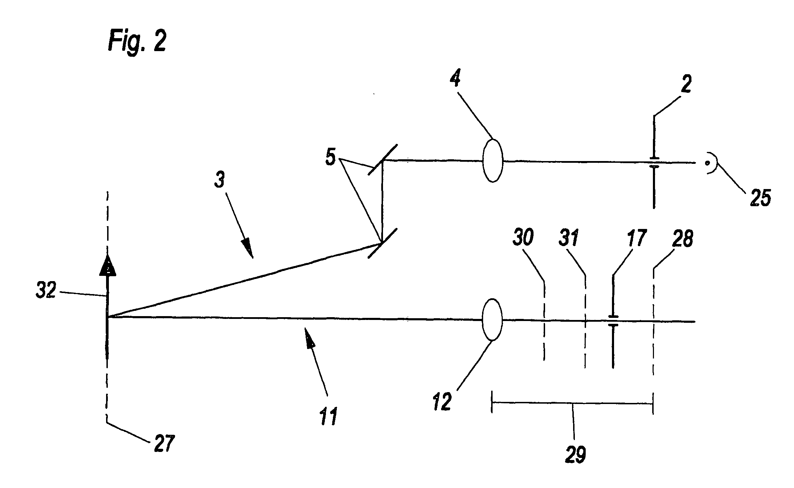

In Fig. 2 ist eine allgemeine Prinzipskizze zu einem abbildenden optischen Gerät mit zumindest abschnittsweise voneinander getrenntem Beobachtungsstrahlengang 11 und Beleuchtungsstrahlengang 3 gezeigt. Die Lichtquelle 25 sendet Licht aus, wobei die Beleuchtungsfeldblende 2 nur für einen gewissen Teil des ausgesendeten Lichts durchlässig ist, während das Licht außerhalb der Blendenöffnung ausgeblendet wird. Der weitere Strahlenverlauf entlang des Beleuchtungsstrahlenganges 3 erfolgt über die Beleuchtungslinsenanordnung 4 (hier als einfache Sammellinse ausgebildet) über die Ablenkprismen 5 zum abzubildenden Gegenstand 32. Der abzubildende Gegenstand 32 sowie die Beleuchtungslinsenanordnung 4 und die Beleuchtungsfeldblende 2 sind hierbei in der Weise angeordnet, daß die Beleuchtungsfeldblende 2 über die Linsenanordnung 4 in die Beleuchtungsbildebene 27 und auf den abzubildenden Gegenstand 32 abgebildet wird. Hierdurch erfolgt eine gezielte und bereichsweise, der Form der Öffnung der Beleuchtungsfeldblende 2 entsprechende Ausleuchtung des abzubildenden Gegenstandes 32. Dabei ist es unerheblich, ob der abzubildende Gegenstand 32 wie in Fig. 2 dargestellt, sich direkt in der Beleuchtungsbildebene 27 befindet, oder, ob der abzubildende Gegenstand in Form eines reellen Zwischenbildes in der Beleuchtungsbildebene 27 vorliegt. Die Abbildung des Gegenstandes 32 in die Beleuchtungsbildebene 27 kann, z.B. wie in Fig. 1 und 3 gezeigt, mittels einer Ophthalmoskopielupe 7 erfolgen. Die Zwischenbildebene 6 aus Figur 1 und 3 liegt hierbei in der Beleuchtungsbildebene 27.

Der wie oben geschildert beleuchtete abzubildende Gegenstand 32 wird über den Beobachtungsstrahlengang 11 und die Beobachtungslinsenanordnung 12 (hier wieder als einfache Sammellinse ausgebildet) auf der Beobachtungsbildebene 28 abgebildet. Beim Stand der Technik ist es hierbei bekannt, in der Beobachtungsbildebene 28 eine Beobachtungsfeldblende 13 (in Fig. 2 nicht dargestellt) anzuordnen. Die Beobachtungsbildweite 29 (definiert als Abstand zwischen Beobachtungslinsenanordnung 12 und Beobachtungsbildebene 28) ist hierbei vom Abstand des zu betrachtenden Gegenstandes 32 von der Beobachtungslinsenanordnung 12 sowie von deren optischen Eigenschaften abhängig. Die Brennebene 30 der Beobachtungslinsenanordnung 12 sowie die Bildebene 31 der Lichtquelle 25 sind in Fig. 2 ebenfalls dargestellt.

Erfindungsgemäß ist nun vorgesehen, mindestens einen Raumfilter 17 außerhalb der Beobachtungsbildebene 28 im Beobachtungsstrahlengang 11 anzuordnen. Hierbei ist es günstig, daß der Raumfilter, welcher hier ebenfalls als Blende ausgeführt ist, auch außerhalb der Brennebene 30 und der Bildebene 31 der Lichtquelle 25 angeordnet ist. Die in Fig. 2 dargestellte Prinzipskizze zeigt hierbei nur eine einfache Ausführungsvariante, bei der nur eine Raumfilterblende vor der Beobachtungsbildebene 28 angeordnet ist. Zusätzlich können, wie in den Fig. 3 und 4 dargestellt, auch noch weitere Raumfilter 17, 18 vor oder hinter der Beobachtungsbildebene 28 im Beobachtungsstrahlengang 11 angeordnet werden. Darüber hinaus wird ebenfalls eine weitere Verbesserung der Filterwirkung erzielt, wenn zusätzlich noch im Beleuchtungsstrahlengang 3 vor und/oder hinter der Beleuchtungsfeldblende 2 weitere Raumfilter 33 angeordnet werden. Hierbei ist es grundsätzlich günstig, daß sowohl alle Raumfilter 17, 18, 33 sowie Feldblenden 2, 13 die selbe Öffnungsform aufweisen. Hierbei kann z.B. eine V-förmige oder auch streifenförmige Ausbildung der Öffnung der Blenden 2, 13, 17, 18, 33 vorgesehen sein.

Das Verwenden von Linsenanordnungen bzw. Linsen mit denselben optischen Eigenschaften als Beleuchtungslinsenanordnung 4 und als Beobachtungslinsenanordnung 12 hat den Vorteil, daß dann die einzelnen Raumfilterblenden 17, 18, 33 und die Feldblenden 2 und 13 einfacher in ein gemeinsames Bauteil 34 zusammengefaßt werden können. Sowohl Beleuchtungslinsenanordnung 4 als auch Beobachtungslinsenanordnung 12 können in einer einfachen Variante als einfache Linse, vorzugsweise Sammellinse, ausgeführt sein. Aus der in Fig. 2 dargestellten Winkelstellung zwischen Beobachtungsstrahlengang 11 und Beleuchtungsstrahlengang 3 ergibt sich, daß der Beobachter des in der Beobachtungsbildebene 28 abgebildeten Bildes nicht von der beleuchtenden Lichtquelle 25 geblendet wird. Durch die Verwendung einer Lichtquelle 25, welche aufgrund eines geeignet geformten Reflektors paralleles Licht aussendet, fallen die Brennebene 30 sowie die Bildebene der Lichtquelle 31 zusammen.

Beim Ophthalmoskop gemäß Fig. 3 sind nun erfindungsgemäß eine weitere vordere Doppelblende 14 und eine weitere hintere Doppelblende 15 als Raumfilter in einer Baueinheit mit der die Feldblenden bildenden Doppelblende 1 angeordnet. Durch diese direkte Kopplung der Doppelblenden 14 und 15 mit der Doppelblende 1 führt das gesamte daraus entstandene Bauteil 34 eine Oszillation aus. Allgemein sollten die Öffnungen der als Raumfilter fungierenden Doppelblenden 14 und 15 eine ähnliche oder gleiche Form, wie die Feldblendenöffnungen 2 und 13 haben. Dies ist in dem in Fig. 3 gezeigten Ausführungsbeispiel gegeben. In diesem Ausführungsbeispiel werden die durch die Doppelblenden 14 und 15 gebildeten Raumfilter mit dem Streifenscanner (allgemein gültig auch für Punktscanner) mitbewegt und sind so im Stande, für jeden Bildstreifen (Bildpunkt) unnötige bzw. unscharf abgebildete und deshalb störende Bildfrequenzen bzw. Beugungsordnungen auszublenden. Dadurch hat jeder Lichtstreifen auf der Retina 10, die für seine Qualität optimale Zahl von Beugungsordnungen.

Das in Fig. 3 gezeigte erfindungsgemäße Ausführungsbeispiel wird aus insgesamt vier Blenden, nämlich einer hinteren 18 und einer vorderen Raumfilterblende 17 im Beleuchtungsstrahlengang 3 und einer hinteren 18 und einer vorderen Raumfilterblende 17 im Beobachtungsstrahlengang 11 gebildet. Allgemein kann jedoch auch mit weniger Blenden eine Raumfilterwirkung erzielt werden.

Der Vorteil der Verwendung einer vorderen 14 und einer hinteren Doppelblende 15 wird anhand des Vergleichs zwischen den Fig. 3 a und 3 b gezeigt. In Fig. 3 a sind schematisch zwei Randstrahlen 19 und 20 dargestellt. Durch die alleinige Verwendung der Raumfilterblende 17 wird nur der äußere Randstrahl 19 blockiert. Der innere Randstrahl 20 hingegen kann ungehindert die Anordnung aus der vorderen Raumfilterblende 17 und der Beobachtungsfeldblende 13 passieren. Verwendet man im Gegensatz hierzu eine Anordnung aus einer vorderen Raumfilterblende 17 und einer hinteren Raumfilterblende 18, wie in Fig. 3 b gezeigt, so wird der äußere Randstrahl 19 nach wie vor von der vorderen Raumfilterblende 17 blockiert. Im Gegensatz zu der in Fig. 3 a gezeigten Anordnung wird jedoch hier auch der innere Randstrahl 20 durch die hintere Raumfilterblende 18 blockiert.

Neben der Anwendung der erfindungsgemäßen Raumfilter in Kombination mit dem in Fig. 1 gezeigten Ophthalmoskop bzw. Streifenscanner gemäß der PCT-Schrift WO 97/15855 gibt es noch zahlreiche weitere Anwendungen für die erfindungsgemäßen Raumfilter. Die erfindungsgemäßen Raumfilter sind somit nicht auf die bereits gezeigten Anwendungsbeispiele beschränkt.

Ein weiteres Anwendungsbeispiel der erfindungsgemäßen Raumfilter wird in Fig. 4 gezeigt. Hier ist ein Auflichtmikroskop - dargestellt mit Objekt 21, Mikroskopobjektiv 22 sowie Spiegel 23, halbdurchlässigem Spiegel 24, Lichtquelle 25 (z.B.

Halogenbeleuchtung) und CCD-Kamera 26 - mit einer Blende für Beleuchtung und Beobachtung erfindungsgemäß mit einem Raumfilter gekoppelt. Das Grundprinzip der beiden Strahlengänge sowie der oszillierenden Doppelblende 1' entspricht dem in Fig. 1 gezeigten Grundprinzip eines Ophthalmoskopes und soll daher nicht mehr gesondert erläutert werden. Analoge Bauteile mit gleicher Funktion werden in Fig. 4 mit denselben, jedoch mit Strichen versehenen Zahlen bezeichnet. Wie in Fig. 3 ist bei dieser Ausführungsform eines Auflichtmikroskops die Doppelblende 1' mit einer als Raumfilter fungierenden vorderen 14' und einer hinteren Doppelblende 15' gekoppelt. Beide führen eine Oszillation in der Schwingungsebene 16' aus. Durch diese erfindungsgemäße Anordnung der als Raumfilter fungierenden Doppelblenden 14' und 15' werden ebenfalls aberrierende Strahlen aus dem Beobachtungsstrahlengang 11' und dem Beleuchtungsstrahlengang 3' herausgefiltert.

## Patentansprüche

1. Abbildendes optisches Gerät mit einer Lichtquellenanordnung und einem von einem Beobachtungsstrahlengang zumindest abschnittsweise getrennten Beleuchtungsstrahlengang, wobei im Beleuchtungsstrahlengang eine Beleuchtungslinsenanordnung und im Beobachtungsstrahlengang eine Beobachtungslinsenanordnung vorgesehen ist, wobei im Beleuchtungsstrahlengang eine Beleuchtungsfeldblende in einem Abstand von der Beleuchtungslinsenanordnung angeordnet ist und ein abzubildender Gegenstand in der Beleuchtungsbildebene liegt, in die die Beleuchtungslinsenanordnung die Beleuchtungsfeldblende abbildet und wobei die Beobachtungslinsenanordnung den abzubildenden Gegenstand in die Beobachtungsbildebene abbildet, **dadurch gekennzeichnet, dass** mindestens ein, insbesondere durch eine Blende gebildeter, Raumfilter im Beobachtungsstrahlengang außerhalb der Beobachtungsbildebene angeordnet ist, wobei der bzw. die Raumfilter (17, 18) und die Beleuchtungsfeldblende (2) sowie eine gegebenenfalls vorhandene Beobachtungsfeldblende (13) in einem festen geometrischen Zusammenhang zueinander - vorzugsweise oszillierend - beweglich gelagert sind.

2. Abbildendes optisches Gerät nach Anspruch 1, **dadurch gekennzeichnet, dass** mindestens ein Raumfilter (17, 18) außerhalb der Brennebene (30) der Beobachtungslinsenanordnung (12) angeordnet ist.

3. Abbildendes optisches Gerät nach Anspruch 1 oder 2, **dadurch gekennzeichnet, dass** mindestens ein Raumfilter (17, 18) außerhalb der Bildebene (31) der Lichtquelle (5) angeordnet ist.

4. Abbildendes optisches Gerät nach einem der Ansprüche 1 bis 3, **dadurch gekennzeichnet, dass** ein Raumfilter (17) vor und ein Raumfilter (18) hinter der Beobachtungsbildebene (28) im Beobachtungsstrahlengang (11) angeordnet sind.

5. Abbildendes optisches Gerät nach einem der Ansprüche 1 bis 4, **dadurch gekennzeichnet, dass** zwei Raumfilter (17) vor oder hinter (18) der Beobachtungsbildebene (28) im Beobachtungsstrahlengang (11) angeordnet sind.

6. Abbildendes optisches Gerät nach einem der Ansprüche 1 bis 5, **dadurch gekennzeichnet, dass** eine Beobachtungsfeldblende (13) in der Beobachtungsbildebene (28) im Beobachtungsstrahlengang (11) angeordnet ist.

7. Abbildendes optisches Gerät nach einem der Ansprüche 1 bis 6, wobei die Beobachtungsbildebene in einer Beobachtungsbildweite von der Beobachtungslinsenanordnung entfernt ist, **dadurch gekennzeichnet, dass** mindestens ein, vorzugsweise alle, Raumfilter (17, 18) im Beobachtungsstrahlengang (11) weniger als 20 %, vorzugsweise weniger als 10 %, der Beobachtungsbildweite (29) von der Beobachtungsbildebene (28) entfernt sind.

8. Abbildendes optisches Gerät nach einem der Ansprüche 1 bis 7, wobei die Beobachtungsbildebene in einer Beobachtungsbildweite von der Beobachtungslinsenanordnung entfernt ist, **dadurch gekennzeichnet, dass** mindestens ein, vorzugsweise alle, Raumfilter (17, 18) als eine Blende mit einer öffnungsweite zwischen 0,5 % und 1,5 %, vorzugsweise von 1%, der Beobachtungsbildweite (29) ausgeführt ist (sind).

9. Abbildendes optisches Gerät nach einem der Ansprüche 1 bis 8, **dadurch gekennzeichnet, dass** zumindest ein weiterer Raumfilter (33) vor und/oder hinter der Beleuchtungsfeldblende (2) im Beleuchtungsstrahlengang (3) angeordnet ist.

10. Abbildendes optisches Gerät nach einem der Ansprüche 1 bis 9, **dadurch gekennzeichnet, dass** es ein Scanner oder ein Mikroskop oder ein Ophtalmoskop ist.

## Claims

1. An optical imaging apparatus comprising a light source arrangement and an illumination beam path which is separate at least in portion-wise manner from an observation beam path, wherein an illumination lens arrangement is provided in the illumination beam path and an observation lens arrangement is provided in the observation beam path, wherein an illumination field stop is arranged in the illumination beam path at a spacing from the illumination lens arrangement and an object of which the image is to be formed is in the illumination image plane in which the illumination lens arrangement images the illumination field stop and wherein the observation lens arrangement images the object of which the image is to be formed in the observation field plane, **characterised in that** at least one spatial filter formed in particular by a stop is arranged in the observation beam path outside the observation image plane, wherein the spatial filter or filters (17, 18) and the illumination field stop (2) and an optionally present observation field stop (13) are mounted movably relative to each other - preferably oscillatingly - in a fixed geometrical relationship.

2. An optical imaging apparatus according to claim 1 **characterised in that** at least one spatial filter (17, 18) is arranged outside the focal plane (30) of the observation lens arrangement (12).

3. An optical imaging apparatus according to claim 1 or claim 2 **characterised in that** at least one spatial filter (17, 18) is arranged outside the image plane (31) of the light source (5).

4. An optical imaging apparatus according to one of claims 1 to 3 **characterised in that** a spatial filter (17) is arranged in front of the observation image plane (28) in the observation beam path (11) and a spatial filter (18) is arranged behind the observation image plane.

5. An optical imaging apparatus according to one of claims 1 to 4 **characterised in that** two spatial filters are arranged in front of (17) or behind (18) the observation image plane (28) in the observation beam path (11).

6. An optical imaging apparatus according to one of claims 1 to 5 **characterised in that** an observation field stop (13) is arranged in the observation image plane (28) in the observation beam path (11).

7. An optical imaging apparatus according to one of claims 1 to 6 wherein the observation image plane is spaced at an observation image distance from the observation lens arrangement **characterised in that** at least one and preferably all spatial filters (17, 18) in the observation beam path (11) are spaced less than 20% and preferably less than 10% of the observation image distance (29) from the observation image plane (28).

8. An optical imaging apparatus according to one of claims 1 to 7 wherein the observation image plane is spaced at an observation image distance from the observation lens arrangement **characterised in that** at least one and preferably all spatial filters (17, 18) is/are in the form of a stop with an aperture width of between 0.5% and 1.5%, preferably 1%, of the observation image distance (29).

9. An optical imaging apparatus according to one of claims 1 to 8 **characterised in that** at least one further spatial filter (33) is arranged in front of and/or behind the illumination field stop (2) in the illumination beam path (3).

10. An optical imaging apparatus according to one of claims 1 to 9 **characterised in that** it is a scanner or a microscope or an ophthalmoscope.

## Revendications

1. Appareil optique d'imagerie, avec un dispositif de source lumineuse et un trajet de rayons d'éclairement, séparé, au moins par tronçons, d'un trajet de rayons de rayons d'observation, où, dans le trajet de rayons d'éclairement, est prévu un dispositif à lentille d'éclairement et, dans le trajet de rayons d'observation, est prévu un dispositif à lentille d'observation, où dans le trajet de rayons d'éclairement est disposé un diaphragme de champ d'éclairement, à distance du dispositif à lentille d'éclairement, et un objet à imager est situé dans le plan image d'éclairement, dans lequel le dispositif à lentille d'éclairement image le diaphragme de champ d'éclairement, et où le dispositif à lentille d'éclairement image l'objet à imager dans le plan image d'observation, **caractérisé en ce qu'**au moins un filtre spatial (17, 18), en particulier formé par un diaphragme, est disposé dans le trajet de rayons d'observation, hors du plan image d'observation, où le ou les filtres spatial/spatiaux et le diaphragme de champ d'éclairement (2), ainsi qu'un diaphragme de champ d'observation (13) présent le cas échéant, sont montés de façon mobile - de préférence de façon oscillante - l'un par rapport à l'autre, selon une relation géométrique fixe.

2. Appareil optique d'imagerie selon la revendication 1, **caractérisé en ce qu'**au moins un filtre spatial (17,18) est disposé hors du plan focal (30) du dispositif à lentille d'observation (12).

3. Appareil optique d'imagerie selon la revendication 1 ou 2, **caractérisé en ce qu'**au moins un filtre spatial (17, 18) est disposé hors du plan image (31) de la source lumineuse (5).

4. Appareil optique d'imagerie selon l'une des revendications 1 à 3, **caractérisé en ce qu'**un filtre spatial (17) et un filtre spatial (18) sont disposés devant et derrière le plan image d'observation (28), en observant dans le trajet de rayons d'observation (11).

5. Appareil optique d'imagerie selon l'une des revendications 1 à 4, **caractérisé en ce que** deux filtres spatiaux (17) sont disposés devant ou derrière (18) le plan image d'observation (28), en observant dans le trajet de rayons d'observation.

6. Appareil optique d'imagerie selon l'une des revendications 1 à 5, **caractérisé en ce qu'**un diaphragme de champ d'observation (13) est disposé dans le plan image d'observation (28) en observant dans le champ de rayons d'observation (11).

7. Appareil optique d'imagerie selon l'une des revendications 1 à 6, où le plan image d'observation est disposé à une distance d'image d'observation vis-à-vis du dispositif à lentille d'observation, **caractérisé en ce qu'**au moins un, de préférence tous les filtres spatiaux (17, 18), dans le trajet de rayons d'observation (11), sont éloignés de moins de 20 %, de préférence de moins de 10 %, de la distance d'image d'observation (29) vis-à-vis du plan image d'observation (28).

8. Appareil optique d'imagerie selon l'une des revendications 1 à 7, où le plan image d'observation est éloigné à une distance d'image d'observation vis-à-vis du dispositif à lentille d'observation, **caractérisé en ce qu'**au moins un, de préférence tous les filtres spatiaux (17, 18), est (sont) réalisé(s) sous forme de diaphragme, avec une largeur d'ouverture entre 0,5 % et 1,5 %, de préférence de 1 % de la distance d'image d'observation (29).

9. Appareil optique d'imagerie selon l'une des revendications 1 à 8, **caractérisé en ce qu'**au moins un autre filtre spatial (33) est disposé devant et/ou derrière le diaphragme de champ d'éclairement (2), en observant dans le trajet de rayons d'éclairement (3).

10. Appareil optique d'imagerie selon l'une des revendications 1 à 9, **caractérisé en ce qu'**il est un scanneur, ou un microscope, ou un ophtalmoscope.
